# EUROPEAN PATENT SPECIFICATION

(11) **EP 3 763 397 B1**
(45) Date of publication and mention of the grant of the patent: **12.07.2023**
(21) Application number: 20194225.7
(22) Date of filing: 30.08.2013
(51) Int. Cl.: A61L 15/42, A61L 15/26

(54) **HEMOSTATIC FOAM**
BLUTSTILLENDER SCHAUMSTOFF
MOUSSE HÉMOSTATIQUE

(30) Priority: 31.08.2012 NL 2009400
(43) Date of publication of application: 13.01.2021
(62) Divisional of application: 13759876.9
(73) Proprietor: Stryker European Operations Holdings LLC, Kalamazoo, MI 49002 (US)
(72) Inventor: CHANDRASHEKHAR-BHAT, Bhushan, Kalamazoo, MI 49002 (US); DE GRAAF, Robbert Arnold, Kalamazoo, MI 49002 (US)
(74) Representative: Röthinger, Rainer

(56) References cited:
- WO-A2-2008/036225
- WO-A2-2010/137981
- US-A1- 2010 063 434
- HO CHEON LEE ET AL: "Preparation and acid dye adsorption behavior of polyurethane/chitosan composite foams", FIBERS AND POLYMERS, vol. 10, no. 5, 1 October 2009 (2009-10-01), pages 636-642, XP055061613, ISSN: 1229-9197, DOI: 10.1007/s12221-010-0636-1

## Description

The invention is directed to a hemostatic foam, to a process for preparing a hemostatic foam, and to said foam for use as nasal dressing.

Hemostasis is a process in the body of humans and animals that causes the bleeding of wounds, *e.g*. damaged blood vessels, to stop. Hemostasis is of fundamental importance for the success of surgical operations as well as subsequent wound healing. A hemostatic foam is intended to produce hemostasis by accelerating the clotting process of blood by applying the foam locally to a bleeding surface.

Chitosan and chitosan salts are known to act as hemostatic agents when applied topically. Chitosan is a polysaccharide comprising D-glucosamine units (deacetylated units) and N-acetyl-D-glucosamine units (acetylated units). When applied as a hemostatic agent, chitosan bonds with platelets and red blood cells to form a gel-like clot which seals a bleeding vessel.

Hemostatic dressing comprising chitosan are known in the art. For example, hemostatic dressings comprising a chitosan coating are known from J. Barnard and R. Millner, "A Review of Topical Hemostatic Agents for Use in Cardiac Surgery", The Annals of Thoracic Surgery, 2009. Further, wound dressings made of chitosan acetate are known from Azad et al ("Chitosan Membrane as a wound-healing dressing: characterization and clinical application", 2004, Wiley InterScience), which describe a method for preparing a membranes from chitosan acetate by allowing a mixture of chitosan and acetic acid to settle. The resulting membrane was found to be suitable as a wound dressing and promotes efficient adherence, hemostasis, healing and re-epithelialization of the wound.

WO2010/137981 A2 discloses a biodegradable hemostatic foam comprising a polymer blend of a water- soluble polymer and phase-separated polyurethane, comprising an amorphous segment and a crystalline segment, wherein at least said amorphous segment comprises a hydrophilic segment.

A disadvantage of the hemostatic chitosan dressings known in the art is that large amounts of chitosan hemostatic agents are required to obtain the advantageous hemostatic effects associated with chitosan. A disadvantage of using high amounts of chitosan in hemostatic dressings is that it may damage white blood cells present in blood. In particular, it was found in an *in vivo* hematology assay (see example 3) that an increased amount of chitosan hemostatic agent resulted in a decrease of white blood cell concentration in the blood.

A further disadvantage of the hemostatic chitosan dressings known in the art is that they show poor mechanical properties, for example with respect to strength and compressibility. For example, chitosan dressings quickly disintegrate after having absorbed blood, which decreases the healing potential of the dressings.

An object of the invention is to provide a foam showing both good hemostatic and mechanical properties.

In particular, it is an object of the invention to provide a hemostatic foam that comprises low amounts of hemostatic agent, in particular chitosan hemostatic agent, while still showing the desirable hemostatic properties of chitosan.

A further object of the invention is to provide a process for making such a hemostatic foam.

At least one of these objects has been met by providing a hemostatic foam for packing antrums or other cavities of the human or animal body comprising a blend of a chitosan hemostatic agent and a phase-separated polymer that provides the foam with a porosity of 85-99% and a foam density of 0.01-0.2 g/cm3, wherein the foam density is calculated as the polymer mass per volume unit foam, wherein said phase-separated polymer comprises an amorphous segment and a crystalline segment, wherein at least said amorphous segment comprises a hydrophilic segment; and wherein the amount of chitosan hemostatic agent is 5-35 wt. % of the total weight of the foam.

The inventors found that a foam according to the invention, when applied to a bleeding surface, showed enhanced hemostatic activity in the blood, thereby increasing the coagulation speed of the blood. Surprisingly, it was found that low concentrations of chitosan hemostatic agent (5-35 wt.%) are sufficient to provide to foam with a good hemostatic activity. Even more surprisingly, the enhanced hemostatic activity was found to be almost independent of the concentration of chitosan hemostatic agent.

Without wishing to be bound by theory, it is believed that the interaction of the polymer, which is a phase-separated polymer as defined in the claims, and the chitosan hemostatic agent in the foam leads to a synergetic effect with respect to the hemostatic activity. Polymer foams having a porosity of 85-99% and a foam density of 0.01-0.2 g/cm³ are capable of absorbing blood, but generally have no hemostatic activity of itself. Hemostatic agents are capable of hemostatic activity when applied to a wound in a sufficient amount. The inventors found that a foam comprising a blend of a hemostatic agent and a polymer that provides the foam with the above-defined porosity and foam density was found to be capable of rapid blood coagulation (as shown by the Lee-White test in the experimental section below), even when the hemostatic agent is present in the foam at low concentrations. The polymer is believed to provide the foam with such mechanical (compressibility, strength, and absorption), structural (area/volume ratio) and chemical properties (hydrophilic/hydrophobic) that the hemostatic agent shows an enhanced hemostatic activity. In particular porosity and density are considered important to achieve this enhanced hemostatic activity. This makes it possible to include the hemostatic agent in very low concentrations in the foam.

Furthermore, the presence of hemostatic agent was found not to negatively influence the mechanical, structural and/or chemical properties of the foam, which are mainly determined by the polymer. In particular, it was found that the foam may essentially retain its compression strength when saturated with blood. This makes the foam particular suitable as a hemostatic foam or dressing.

It is further believed, again without wishing to be bound by theory, that the hemostatic foam of the present invention, when applied to a bleeding surface, manages to locally increase the concentration of platelets and other hemostasis stimulating compounds by absorbing water from the blood. The increased concentration then promotes coagulation of the blood and contributes to quick and efficient hemostasis.

The term "blend" as used herein refers to a mixture of two or more different polymers, *i.e.* the polymer (base polymer) and the chitosan hemostatic agent (hemostatic polymer). As used herein, the term "blend" and "polymer blend" can be used interchangeably. The mixture of polymers may be macroscopically homogeneous. The polymers in a polymer blend are preferably randomly distributed throughout the blend. In a polymer blend, cross-linking between the two or more different polymers is typically avoided. Accordingly, the degree of cross-linking of the polymers in the polymer blend is typically 0.01 or lower, more preferably 0.001 or lower, most preferably about 0. The degree of cross-linking is a well-known parameter and may also be referred to as cross-linking density. It is a measure of the amount of bondings between two polymer chains and may in particular refer to the number of bondings formed in and between the polymers of the blend per total amount of monomer units of the two or more polymers present in the blend.

The term "foam density" as used herein refers to the density of foam, calculated as the polymer mass per volume unit of foam. The mass of the hemostatic agent present in the foam is disregarded when calculating the foam density.

The foam of the invention has a porosity of 85-99% and a foam density of, for example, 0.03-0.07 g/cm³. Such values for the porosity and density contribute to the enhanced hemostatic activity, as described above, and also provide the foam with good liquid (*e.g*. water or blood) absorbing properties. Preferably, the foam has a polymer porosity of 92-98%, even more preferably 95-98%. As described above, a combination of these values for the porosity and density was found to increase the hemostatic effect of chitosan hemostatic agents present in the foam. Most preferably the foam has a porosity of 95-98% and a foam density of 0.03-0.7 g/cm³.

The polymer present in the foam of the invention is a polymer as defined in the claims, which is capable of forming a foam having the same porosity and density as specified for the hemostatic foam of the invention. Such polymers are known in the art and the skilled person will have no difficulty selecting a suitable polymer. According to the invention, the polymer is a phase-separated polymer as described in detail below. The use of such a polymer does not only result in the desirable porosity and density as defined above, but also provides the foam with good compressibility, which means that the foam retains its structure (in particular its compression strength) when having absorbed or being saturated with a liquid such as blood.

The mechanical, structural and chemical properties of the foam are mainly determined by the polymer present in the foam. The chitosan hemostatic agent does essentially not influence these properties, except for providing it with hemostatic activity. This is advantageous, because the present invention thus provides for a way to control and adjust the mechanical, structural and chemical properties of a hemostatic foam by selecting a suitable polymer.

As described above, small amounts of hemostatic agent are sufficient to provide the foam with desirable hemostatic properties. Since the hemostatic activity of the foam of the invention is almost independent on hemostatic agent, high concentrations are generally neither required nor preferred.

The chitosan hemostatic agent is preferably present in the foam in the form of particles, in particular polymeric particles. Examples of suitable particles are amorphous, crystalline and gel-like particles. The hemostatic agents may also be liquid, in particular when highly viscous. In case of hemostatic particles, the particles may have a size from 1-1000 µm. Preferably, particles are smaller than 150 µm. In particular good results have been obtained using particles of 5-90 µm. Small particles have a number of advantages. First, the structure of the foam is less influenced by the presence of small particles than large particles. Second, small hemostatic particles have a smaller tendency to aggregate than large particles. Furthermore, a good dispersion may be obtained using small particles. Lastly, small particles do not settle down when preparing the foam, such that a homogeneous distribution within the foam may be achieved if desirable.

The hemostatic particles may be any suitable shape but are preferably roughly spherical. The particles are preferably solid. Suitable solid particles to be used are generally insoluble and hydrophilic.

The particles may be preferentially distributed at the boundaries of the foam, or homogeneously throughout the foam, or as a gradient within the foam.

The hemostatic agent may be randomly distributed within the foam.

The term "chitosan hemostatic agent" as used herein refers to chitosan or a salt or derivative thereof. Good results have been obtained using chitosan acetate. Further examples of suitable chitosan salts are chitosan esters of glutamate, succinate, phtalate or lactate, chitosan derivatives comprising one or more carboxymethyl cellulose groups, carboxymethyl chitosan. Other suitable examples of chitosan derivates are chitosan with quarternary groups (like N-thrimethylene chloride, N-trimethylene ammonium). Also, bioactive excepients such as calcitonin or 5-methylpyrrolidinone can be used.

As mentioned above, chitosan is a polysaccharide comprising D-glucosamine units (deacetylated units) and N-acetyl-D-glucosamine units (acetylated units). Chitosan can be prepared from chitin by deacetylating at least part of the N-acetyl-D-glucosamine in chitin (poly-N-acetyl-D-glucosamine) by hydrolysis. The ratio of D-glucosamine units and N-acetyl-D-glucosamine units in chitosan is typically expressed as the degree of deacetylation. The degree of deacetylation is defined as the percentage of glucosamine units in chitosan that are not acetylated. This percentage thus corresponds to the molar percentage of deacetylated units present in chitosan.

Chitosan as used in the invention may have a degree of deacetylation of 1-100 mol%, more preferably 5 - 50%, even more preferably 10-25 mol%. Chitosan having a relatively low degree of deacetylation generally shows a high hemostatic effect. However, pure chitin (0% deacetylation) does not have sufficient hemostatic. The above values also apply to chitosan present in chitosan salts, as well as to chitosan derivatives (which have acetylated and deacetylated units just like chitosan itself).

Suitable chitosan salts are those wherein the chitosan ion has a net positive charge. Accordingly, suitable chitosan salts may be salts consisting of a chitosan cation and a counter anion. For example, the chitosan hemostatic agent may be a salt of chitosan with an organic acid, in particular with a carboxylic acid such as succinic acid, lactic acid or glutamic acid. Chitosan salts may for example be selected from the group consisting of nitrate, phosphate, glutamate, lactate, citrate, acetate and hydrochloride salts of chitosan.

In general, a chitosan derivative is a chitosan molecule wherein one or more of the hydroxyl groups and/or the amine group present in chitosan has been substituted. For example, the one or more hydroxyl groups may be substituted to obtain an ether or ester. The amine group may be substituted to obtain an amino group, although this generally results in a decrease in hemostatic activity. Therefore, the amine groups of chitosan are preferably unsubstituted.

The chitosan hemostatic agent used in the invention preferably comprises or is preferably derived from chitosan originating from animals, plants or shellfish. These sources give similar good results with respect to the hemostatic effects described above. Furthermore, synthetic chitosan may also be used.

The hemostatic agent used in the present invention may have a molecular weight in the range of about 1-1000 kDa. The molecular weight of chitosan used in the invention is preferably in the range of 10-100 kDa, most preferably 30-80 kDa.

The polymer present in the foam of the invention may be selected from the list consisting of polyesters, polyhydroxyacids, polylactones, polyetheresters, polycarbonates, polydioxanes, polyanhydrides, polyurethanes, polyester(ether)urethanes, polyurethane urea, polyamides, polyesteramides, polyorthoesters, polyaminoacids, polyphosphonates, polyphosphazenes and combinations thereof. The polymers may also be chosen from copolymers, mixtures, composites, cross-linking and blends of the above-mentioned polymers.

Preferably, the polymer is biodegradable. The term "biodegradable" as used herein, refers to the ability of a polymer to be acted upon biochemically in general by living cells or organisms or part of these systems, including hydrolysis, and to degrade and disintegrate into chemical or biochemical products.

The polymer present in the hemostatic foam of the invention is a phase-separated polymer comprising an amorphous segment and a crystalline segment, wherein at least said amorphous segment comprises a hydrophilic segment. Such a polymer is described in WO-A-2004/062704. These polymers were found to show a particular good enhanced hemostatic effect in combination with hemostatic agents. Surprisingly, the mechanical properties (*e.g.* compressibility, strength, and absorption), structural properties (*e.g*. area/volume ratio) and chemical properties (hydrophilic/hydrophobic) of foams prepared from such a polymer were essentially unaltered by inclusion of the hemostatic agent by blending. This makes it possible to provide hemostatic foams having very desirable mechanical, structural and chemical properties.

As described in WO-A-2004/062704, the amorphous segment of the phase-separated polymer must comprise a hydrophilic segment. This amorphous segment, also called the amorphous phase in the art, is amorphous when applied to a bleeding surface, *i.e.* when wet, despite the fact that it may comprise a crystalline polyether. This means that, in the dry state, said crystalline polyether may provide the amorphous phase of the polymer with partially crystalline properties. The performance of the foam when applied to a bleeding surface determines the characteristics of the foam: when applied to a bleeding surface, the foam of the invention is comprised of an amorphous hydrophilic soft segment or phase and a crystalline hard segment or phase.

Hydrophilic groups may also be present in the hard segment of the phase-separated polymer, but the presence of hydrophilic groups in the hard segment should not result in immediate disintegration of the foam when placed in contact with fluids. Essentially, the crystalline hard segment or phase must provide the foam with rigidity, keep the foam intact and prevent swelling of the foam when placed in contact with fluids.

Preferably, the phase-separated polymer is a polymer comprising one or more urea, urethane, amide, carbonate, ester or anhydride link. More preferably, the phase-separated polymer is a polyurea, polyamide or polyurethane, most preferably a polyurethane.

The term "phase-separated polyurethane" as used herein, refers to a polymer comprising soft (amorphous) segments, as well as hard (crystalline) segments, the phase-separated morphology being manifest when the foam prepared from such a polymer is applied to a bleeding surface of a human or animal body for a sufficient period of time. Also, a phase-separated polymer being placed under temperature conditions comparable to the human or animal body exhibits said phase-separated morphology.

A phase-separated polyurethane is characterised by the presence of at least two immiscible or partly miscible phases with a different morphology and different thermal state at normal environmental conditions. Within one material a soft rubbery amorphous phase and a hard crystalline phase (at a temperature above the glass transition temperature of the amorphous phase and below the melting temperature of the crystalline phase) may be present or a hard glassy amorphous phase and a hard crystalline phase (at a temperature below the glass transition temperature of the amorphous phase). Also at least two amorphous phases can be present, *e.g*. one hard glassy and one soft rubbery phase. Even above the melting temperature, the liquid and rubbery phases can still be immiscible. More in particular, when a polyurethane has an amorphous phase and a crystalline phase, which two phases are immiscible with each other, the polyurethane is said to be phase-separated. The presence of immiscible phases (amorphous and crystalline) may be suitably determined by the use of a *e.g.* (modulated) differential scanning calorimetry (DSC).

The phase separated morphology is essential for the mechanical properties of the foams. Both phases contribute to the unique properties of the material of the present invention. The soft, amorphous phase is responsible for the flexible and elastic behavior. The hard, crystalline phase is responsible for the hardness and strength of the material. The (semi) crystalline hard segments undergo intermolecular crystallization and behave as physical cross-links and knot the soft segments in a three dimensions network structure. Because of this, microphase separation may appear where the hard crystalline and the soft amorphous segments can form a cocontinuous two-phase system. Cocontinuous microstructures are characterized by having both phases interpenetrating each other in three dimensions. In a cocontinuous morphology all the hard areas are connected with each other. Due to this morphology the foam has the ability to essentially maintain its compression strength upon blood absorption.

The term "amorphous" as used herein, refers to segments present in the polyurethane of the invention with at least one glass transition temperature below the temperature of the bleeding surface and may also refer to a combination of an amorphous and crystalline segment which is completely amorphous when applied to a bleeding surface. The glass transition temperature may be determined with the use of a (modulated) differential scanning calorimeter.

The term "crystalline" as used herein, refers to segments, present in the polyurethane of the invention, that are crystalline when applied to a bleeding surface, that have a melting temperature above the temperature of the bleeding surface.

A "hydrophilic segment" as used herein, refers to a segment comprising at least one, preferably at least two, more preferably at least three hydrophilic groups, which can for instance be provided by C-O-C, or ether, linkages. A hydrophilic segment may thus be provided by a polyether segment. A hydrophilic segment may also be provided by polypeptide, poly(vinyl alcohol), poly(vinylpyrrolidone) or poly(hydroxymethylmethacrylate). A hydrophilic segment is preferably derived from polyalkyleneglycol, such as polyethylene glycol, polypropylene glycol, or polybutylene glycol. The preferred hydrophilic segment is a polyethylene glycol (PEG) segment.

The term "segment" as used herein, refers to a polymeric structure of any length. In the art of polymer technology a long polymeric structure is often referred to as a block, whereas a short polymeric structure is often referred to as a segment. Both these conventional meanings are understood to be comprised in the term "segment" as used herein.

In one embodiment of the foam of the invention, the polymer is a phase-separated, biodegradable polymer of formula (I):

⁅R-Q¹[-R'-Z¹-[R"-Z²-R‴-Z³]ₚ-R"-Z⁴]_{q}-R'-Q²⁆ₙ (I)

wherein R is a polymer or copolymer selected from one or more aliphatic polyesters, polyether esters, polyethers, polyanhydrides, and/or polycarbonates, and at least one R comprises a hydrophilic segment; R', R" and R‴ are independently C₂-C₈ alkylene, optionally substituted with C₁-C₁₀ alkyl or C₁-C₁₀ alkyl groups substituted with protected S, N, P or O moieties and/or comprising S, N, P or O in the alkylene chain; Z¹-Z⁴ are independently amide, urea or urethane, Q¹ and Q² are independently urea, urethane, amide, carbonate, ester or anhydride, n is an integer from 5-500; and p and q are independent 0 or 1.

The soft segment of the polymer of formula (I) is generally represented by R, whereas the remainder of formula (I) generally represents the hard segment of the polymer.

Although Z¹ - Z⁴ may differ from each other, Z¹ - Z⁴ are preferably chosen to be the same. More preferably, Z¹ - Z⁴ are all urethane moieties and the polymer can in such a case be represented by formula (II): 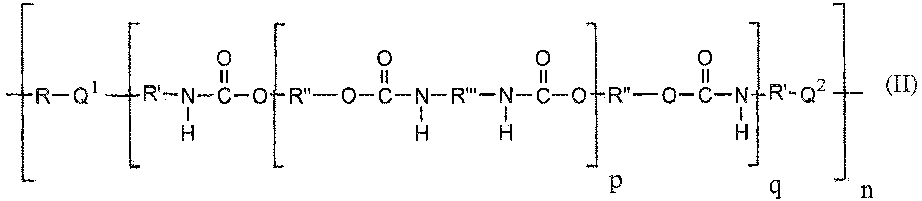 wherein Q¹, Q², R, R', R", R‴, p, q and n are defined as described hereinabove for formula (I).

Q¹ and Q² are chosen independently from each other from the group consisting of urea, urethane, amide, carbonate, ester and anhydride. Preferably, Q¹ and Q² are independently chosen from urethane, carbonate and ester. Although Q¹ and Q² may be chosen to be different kind of moieties, Q¹ and Q² are preferably the same, most preferably both urethane moieties.

Preferably, q=1 in formulas (I) and (II). Thus, the polymer has a hard segment of sufficient length to easily form crystalline domains, resulting in a phase-separated polyurethane. An even more desirable length is obtained for this purpose if both q and p equal 1.

To enhance the phase-separated nature of the polymer, R can be chosen as a mixture of an amorphous and a crystalline segment. For this purpose, R is preferably a mixture of at least one crystalline polyester, polyether ester or polyanhydride segment and at least one amorphous aliphatic polyester, polyether, polyanhydride and/or polycarbonate segment. This may be particularly desirable when q is chosen 0, because the urethane moiety may in such a case be too small to form crystalline domains, resulting in a mixture of both phases, wherein no phase-separation occurs.

According to the present invention, the amorphous segment is comprised in the -R- part of the polymer according to formula (I). The remaining part of the polymer according to formula (I), including the R', R" and R'" units, represents the crystalline segment. The crystalline segment is always a hard segment, while the amorphous segment at least comprises one or more soft segments. R in formula (I) comprises the soft segments, while the remainder of formula 1 typically comprises the hard segments. The soft segments are typically amorphous in the polymer of the invention. The hard segments have a tendency to crystallize, but may be amorphous when not crystallized completely.

R is a polymer or copolymer selected from aliphatic polyesters, polyether esters, polyethers, polyanhydrides, polycarbonates and combinations thereof, wherein at least one hydrophilic segment is provided in at least one amorphous segment of R. Preferably, R is a polyether ester. R can for example be a polyether ester based on DL lactide and ε-caprolactone, with polyethylene glycol provided in the polyether ester as a hydrophilic segment.

R comprises a hydrophilic segment and such a hydrophilic segment can very suitably be an ether segment, such as a polyether segment derivable from such polyether compounds as polyethylene glycol, polypropylene glycol or polybutyleneglycol. Also, a hydrophilic segment comprised in R may be derived from polypeptide, poly(vinyl alcohol), poly(vinylpyrrolidone) or poly(hydroxymethylmethacrylate). A hydrophilic segment is preferably a polyether.

Each of the groups R', R" and R‴ is a C₂ - C₈ alkylene moiety, preferably a C₃ - C₆ alkylene moiety. The alkylene moiety may be substituted with C₁-C₁₀ alkyl or C₁-C₁₀ alkyl groups substituted with protected S, N, P or O moieties and/or comprising S, N, P or O in the alkylene chain. Preferably, the alkylene moiety is unsubstituted (CₙH₂ₙ) or substituted. R', R" and R‴ may all be chosen to be a different alkylene moiety, but may also be the same.

Preferably, R' is an unsubstituted C₄ alkylene (C₄H₈) or an unsubstituted C₆ alkylene (C₆H₁₂). R' may be derived from a diisocyanate of the formula O=C=N-R'-N=C=O, such as alkane diisocyanate, preferably 1,4-butanediisocyanate (BDI) or 1,6-hexanediisocyanate (HDI).

Preferably, R" is an unsubstituted C₄ alkylene (C₄H₈) or an unsubstituted C₃ alkylene (C₃H₆). R" may be derived from a diol of the formula HO-R"-OH, such as 1,4-butanediol (BDO) or 1,3-propanediol (PDO).

Preferably, R‴ is an unsubstituted C₄ alkylene (C₄H₈) or an unsubstituted C₆ alkylene (C₆H₁₂). R' may be derived from a diisocyanate of the formula O=C=N-R‴-N=C=O, such as alkane diisocyanate, preferably 1,4-butanediisocyanate (BDI) or 1,6-hexanediisocyanate (HDI).

A method for preparing phase-separated biodegradable polymer of formula (I) is known in the art, such as for example described in WO-A-2004/062704.

An example of a polymer that can be very suitably used in the blend of the hemostatic foam of the invention is a phase separated polyurethane according to formula (I), wherein R is a polyether ester based on DL lactide and ε-caprolactone, which polyether ester comprises a hydrophilic polyethylene glycol segment; R', R" and R‴ are C₄ alkylene (C₄H₈); Q¹, Q² and Z¹ - Z⁴ are urethane and p=1 and q=1.

Another example that is preferred in accordance with the present invention is a structure wherein R = soft segment based on DL lactide and ε-caprolactone and polyvinylpyrrolidone as the hydrophilic segment.

Another example that is preferred in accordance with the present invention is a structure wherein R = soft segment based on DL lactide and ε-caprolactone and polyvinyl alcohol as hydrophilic segment.

For these latter two structures, R', R" and R‴ are C₄; Q¹, Q² and Z¹ - Z⁴ are urethane; and p =1 and q = 1.

Foam according to the present invention may be "bioresorbable". Bioresorbable refers to the ability of being completely metabolized by the human or animal body. This ability is suitable for certain applications, for example when a hemostatic agent is placed in an antrum or other body cavity.

The hemostatic foam according to the present invention absorbs blood by its hydrophilic nature and porous structure and displays sufficient strength to remain properly positioned during the time of healing of the wound. New tissue may grow into the absorbent foam. After a certain period, which may be controlled by proper selection of the phase-separated polymer used for its manufacture, the hemostatic foam of the invention will degrade to mere residue and may eventually be completely metabolized by the body.

The hemostatic foam is typically capable of absorbing a water volume that is equal to 2-50, preferably 5-40, most preferably 15-25 times its own volume. A good water absorption ensures that the hemostatic foam is capable of absorbing blood. Such a good water absorption is generally provided by the porosity of the foam, which can be achieved by selecting a suitable polymer, such as the phase-separated polymer described above.

The hemostatic foam of the invention preferably has a tensile strength of 5-100, preferably 10-50 MPa. The hemostatic foam of the invention preferably has a modulus of 5-100, preferably 10-75 MPa. The hemostatic foam of the invention preferably has a strain at break of at least 200%, preferably at least 400-800%. Such properties can be obtained by selecting a suitable polymer, such as the phase-separated polymer described above.

The hemostatic foam according to the invention can have any suitable shape, such as a cylinder, a cuboid, a plate, a flake or a cone. Preferably, the hemostatic foam is in the form of a foam pad of 20-250 cm² , preferably 50-150 cm² (e.g. about 10x10 cm) having a thickness of 1-4 cm, preferably 1.5-2 cm.

Good results have further been obtained using porous flakes, which may be porous irregular shaped particles with a size (largest diameter) varying from 0.5 - 4 mm. Porous flakes may be defined in the context of the present invention as containing sufficient pores (holes or other small cavities) so that the structure can hold a liquid or allow it to pass through. The porous flakes are advantageous in stopping excessive bleeding, because these flakes can easily be applied to surfaces which are normally difficult or even impossible to reach for a surgeon.

Once the porous flakes are applied on the bleeding surface, coagulation will start and the flakes will stack together to form one large and dense blood clot. This allows for methods of treatment that were not possible before. If the porous flakes have different sizes and shapes, a better filling of the wound can be obtained, because a higher degree of packing can be obtained.

In a further aspect, the invention is directed to a process for preparing a hemostatic foam according to the invention. This method comprises the steps of
dissolving a phase-separated polymer as defined in claim 1 in a solvent, thus obtaining a polymer solution;
blending a chitosan hemostatic agent with said polymer solution to form a mixture; and
removing said solvent by freeze-drying. The freeze-drying process comprises freezing the polymer mixture and subliming the solvent. The freezing step may be carried out at any suitable temperature to freeze the polymer/particles mixture.

This method is in particular suitable when the hemostatic agent is in the form of particles (hereinbelow referred to as "hemostatic particles" or "particles" for short). In this case, the method of the invention comprises the following steps:
- dissolving at least one synthetic polymer as defined in claim 1 in one or more solvents to form a solution;
- contacting chitosan hemostatic particles with said solution to form a polymer/particles mixture; and
- freeze-drying the polymer/particles mixture by:
   - freezing the polymer/particles mixture; and subsequently
   - subliming the one or more solvents to form a synthetic foam comprising said particles. The particles present in the thus obtained foam were found to have a controlled particle distribution. The method is described in detail in the Dutch application having application number 2007503.

The freeze-drying process comprises freezing the polymer/particles mixture and subliming the solvent. The freezing step may be carried out at any suitable temperature to freeze the polymer/particles mixture.

Once the polymer/particles mixture is frozen, the drying step may be carried out. During the drying step the pressure is lowered and the temperature may be increased such that the solvent sublimes from the frozen polymer/particles mixture. The combination of the freezing and drying processes results in the polymer/particles mixture forming a synthetic foam with a specific distribution of particles. In some embodiments, the temperature increase may be in part from the latent heat of sublimation of the solvent molecules. The drying step may result in up to 90 % and preferably 95 % of the solvent subliming. The entire freeze-drying may last from about 1 h to 24 h or more. Typically, the entire freeze-drying process is performed overnight for a period of about 15 h.

Preferably the mixture is poured into one or more molds prior to freeze-drying. The mold may be a hollow form or cast that allows the polymer/particles mixture to solidify into a particular from. The mold may be any suitable shape and/or size. In some embodiments, multiple molds may be part of a single tray.

Surprisingly it was found that by using the process of the present invention, the distribution of particles within a synthetic foam can be controlled. The particles may be preferentially distributed at the boundaries of the foam, or homogeneously throughout the foam, or as a gradient within the foam.

Furthermore we have found that a homogeneous incorporation of particles into a synthetic foam may be achieved by carrying out the freeze-drying step such that the temperature of the polymer/particles mixture is decreased below the freezing point (crystallization temperature) at a high rate, typically within 10 s.

These cooling rates will depend on the type of solvent or solvents that are used and the speed at which it is possible to sublimate the solvent or solvents from the foam using the freeze drying process. When the temperature of the polymer/particles mixture is lower than the freezing point (crystallization temperature) of the solvent or solvents, the solvent crystallizes. Subliming the solvent or solvents results in a synthetic foam comprising a homogeneous distribution of particles.

Thus, in a further aspect of the process of the present invention, the freeze-drying step comprises:
- freeze-drying the polymer/particles mixture by:
   - freezing the polymer/particles mixture within 60 s; and subsequently
   - subliming the one or more solvents to form a synthetic foam comprising a homogenous distribution of particles.

In an alternate process, we have found that a homogeneous incorporation of particles into a synthetic foam may also be achieved by carrying out a pre-cooling step prior to freeze-drying. The pre-cooling step cools is carried out for a period sufficient to cool the polymer/particles mixture to within + 5 °C from the freezing point of the one or more solvents, and typically takes from about a few seconds to a few minutes.

Thus, in a further aspect of the process of the present invention, the process further comprises pre-cooling the polymer/particles mixture to a temperature within + 5 °C from the freezing point of the one or more solvents prior to freeze-drying.

We have also found that a particle layer at the bottom and sides of the synthetic foam may be achieved by slowly decreasing the temperature of the polymer/particles mixture to the freezing point of the one or more solvents (broad freezing range). Typically the polymer/particles mixture is frozen over a period of 60 s to 600 s. However, the duration of freezing may range about from about 1/100 s to several hours, depending on the material type and weight. Sublimation of the one or more solvents results in a synthetic foam comprising one or more particle layers within the foam. Typically, the particle layers form at the cooling surfaces of a mold, such as the bottom and sides.

In another aspect of the process of the present invention, the freeze drying step comprises:
- freezing the polymer/particles mixture to the freezing point of the one or more solvents within 60 s to 600 s; and subsequently
- drying the polymer/particles mixture by the sublimation of the one or more solvents to form a synthetic foam comprising one or more layers of particles.

It was further found that the rate of decreasing the temperature, whether it is slow or quick, and the starting temperature of the process are all dependent on the freezing point of the solvent. However, the final temperature is not critical, it is only necessary that the foam is frozen.

The process of the present invention is advantageous because by simply changing the temperature profile we are able to regulate the distribution of particles inside the synthetic foam. Further, we have found that due to the properties of the synthetic polymer material used, the particles adhere to the foam. This has the advantage that no binding agent is required in the foam.

Further, the specific distribution of the particles at bottom or side surface or throughout the foam could be advantageous in different applications. For example a foam comprising a bottom layer of particles which are hemostatic in nature, may be used to arrest bleeding almost immediately. Alternatively a foam comprising a homogeneous distribution of particles may be advantageously used in both blood clotting and blood absorption.

Solvents suitable to be used in the process of the present invention are polar solvents which have freezing points in the range of about 0-50 °C. Such solvents may be removed by freeze drying. Such suitable solvents include organic solvents such as acetic acid, benzene, cyclohexane formic acid, nitrobenzene, phenol, 1,4-dioxane, 1,2,4-trichlorbenzene, dimethylsulphoxide (DMSO) and combinations thereof. Preferably the solvent used is 1,4-dioxane.

Surprisingly we have found that by using solvents which are immiscible a synthetic foam with a specific hierarchy in its structure may be created using the process of the present invention. Water in particular may also be used as a suitable solvent in combination with at least one organic solvent to form such an immiscible solution.

The polymer used in the method of the invention is the phase-separated polymer described above.

The polymer may be dissolved in a solvent to form a solution with a polymer concentration of about 2-10 wt. %.

We have also found that the size of the particle used also affects their distribution within the synthetic foam. The use of ultra fine particles in the process of the present invention leads to a good particle distribution throughout the foam and minimizes particle aggregation. The use of larger sized particles, however, is less desirable since this can lead to an increased possibility of coagulation or agglomeration of the particles in the foam. The coagulation of particles in the foam is can result in the foams becoming brittle which would make them unsuitable for use.

The particles used in the method of the invention are the hemostatic agent in particle form described above for the hemostatic foam.

Surprisingly we have found that even when particles lighter than the solvent or solvents are used in the process of the present invention, the particles do not rise to the top as one would expect, instead the particles form a layer underneath the foam

We have also found that a synthetic foam with a well-dispersed particle distribution may be obtained if a partly frozen polymer/particles mixture is heated to just above the freezing point of the polymer/particles mixture and then re-frozen. Subliming the solvent from the frozen polymer/particles mixture results in a synthetic foam with a homogenous distribution of particles. Preferably the particle sizes are small, from about 1-150 µm. In this embodiment the process is not dependent on the freezing temperature of the solvent.

In another embodiment of the process of the present invention, the freeze-drying step comprises:
- freezing at least once the polymer/particles mixture to form a partly frozen polymer/particles mixture; increasing the temperature at least once above the freezing point of the one or more solvents to melt the partly frozen polymer/particles mixture; and decreasing the temperature to re-freeze the polymer/particles mixture; and subsequently
- drying the polymer/particles mixture by sublimation of the one or more solvents to form a synthetic foam comprising a homogenous distribution of particles.

The porosity of the foams produced is 85-99 %, preferably 92-98 %, more preferably 95-98 %.

Suitable shapes of the foam prepared according to the process of the present invention include but are not limited to a rectangular, cylinder, a cuboid, a plate, a flake or a cone.

In yet a further aspect, the present invention is directed to the foam of the invention for use as nasal dressing, e.g., to arrest bleeding in surgical interventions or other injuries. The foam may also be used in general surgery, in oral or dental surgery (*e.g.* extraction of teeth), orthopedic surgery; vascular surgery; neurosurgery; lung surgery; surgery of large abdominal organs and surgery of ear, nose or throat (ENT). The hemostatic foam of the invention is also suitable for packing antrums or other other cavities of the human or animal body, such as for example the nasal cavity or the outer ear. A further application of the hemostatic foam is use as an implant material, in particular for use in soft tissue repair or as a drug delivery vehicle. The hemostatic foam may also be used as a drain, *e.g.* a nasal drain.

The present invention will now be illustrated by means of the following examples.

### Example 1: Preparation of the Hemostatic Foam

Different hemostatic foams were prepared by the following method. Chitosan hemostatic particles having a particle size of 20-30 µm were mixed with a polymer solution of 0.50 g of polymer in solvent dioxane at room temperature. The resulting polymer/particle mixture was freeze-dried by first freezing it to a temperature of 0 to -20°C and then subliming the solvent by freeze drying it in vacuum with a temperature gradient from -20°C to room temperature over time.

Foams were prepared with either chitosan or chitosan acetate as the chitosan hemostatic agent.

The amount of chitosan hemostatic particles added to the polymer solution varied from 120 to 840 mg.

The polymer used was a phase-separated polyurethane polymer according to formula II, wherein R represents PEG and R' represents a C4 alkylene.

The thus obtained foam had a chitosan or chitosan acetate concentration varying from 5-35 mg per cm³, which corresponds to a weight percentage of 20-65 wt.%.

The foam showed good mechanical properties. In particular, it had a porosity of 95-97% and a density of 0.03-0.07 g/cm³.

### Example 2: Effect of Chitosan Concentrations on Hemostatic Effect

Different hemostatic foams were prepared in a similar way as in Example 1 and tested for its hemostatic activy using the Lee-White method.

The Lee-White test determines if the clotting time of whole human blood is affected by the presence of the test article. The sample is exposed directly to blood and the time taken for the blood to clot is measured. The clotting time of the blood with test material is compared to the time taken for untreated blood to clot. Whole human blood forms a clot in between 8-15 minutes. The presence of a sample may enhance or decrease the clotting time.

The Lee-White method was performed by a GLP accreditated firm. The foams tested were those obtained in Example 1. The results are shown in Tables 1 and 2.

**Table 1: Results Lee-White Test**

| Control / Polymer used | Type of chitosan hemostatic agent | molecular weight | Chitosan hemostatic agent concentration (mg/cm³) | Clotting time (min) |
|---|---|---|---|---|
| Negative (polyethylene) | NA | NA | NA | 27.8 |
| Untreated (blood only) | NA | NA | NA | 31.7 |
| polyurethane | Chitosan acetate | low | 5 | 7.0 |
| polyurethane | Chitosan acetate | low | 10 | 6.8 |
| polyurethane | Chitosan acetate | low | 25 | 6.7 |
| polyurethane | Chitosan acetate | low | 35 | 7.0 |
| polyurethane | Chitosan acetate | high | 5 | 6.8 |
| polyurethane | Chitosan acetate | high | 10 | 7.5 |
| polyurethane | Chitosan acetate | high | 25 | 7.3 |
| polyurethane | Chitosan acetate | high | 35** | 7.5 |
| polyurethane | Chitosan | * | 10 | 7.2 |

| | | | | |
|---|---|---|---|---|
| * chitosan derived from shrimp was used as the hemostatic agent ** not claimed | | | | |

**Table 2: Summarized Results Lee-White Test**

| Samples | | Clotting time (min) |
|---|---|---|
| Control | Negative | 27.8 |
| Control | Untreated | 31.7 |
| Test articles | | 6.8 to 7.5 |

From Tables 1 and 2 it can be seen that there is enhanced decrease in the clotting time of blood.

In all experiments, hemostase was achieved in 6.5-7.5 minutes, which is about 75 % faster compared to the hemostase achived by the blood itself. The hemostatic effect was for all agents at all concentrations about the same. The variance shown was attributed merely to physiological differences in the blood.

### Example 3: Effect of Foams on Cellular Components in Human Blood

In this example, the hematology of different hemostatic foams was measured using the in-vitro haematology assay. The in-vitro haematology assay is designed to ensure that the test article does not adversely affect the cellular components of the blood. The test article or its extracts is incubated in whole human blood, a test system that is recommended by ISO guidelines, and following parameters are measured: complete blood count (CBC), haematocrit, platelet count, hemoglobin, mean cell hemoglobin, hemoglobin concentration and mean cell volume. The test is carried out in triplicate and the results after exposure to the test article are statistically compared to those of untreated and negative controls (if p≥0.05, the test article passes).

The test articles that were used were those obtained in Example 1. As an untreated control, blood was used. As a negative control, polyethylene plastic was used.

No significant differences were observed between test samples when compared to negative (polyethylene plastic) and untreated samples (only blood), as shown in Table 3.

**Table 3**

| Samples | | WBC's* (10³/µL) | RBC's* (10⁶/µL) | Hemoglobin (g/dL) | Hematocrit (%) | Platelet's (10³/µL) |
|---|---|---|---|---|---|---|
| Control | Negative | 3.44 | 3.86 | 11.6 | 34,3 | 128 |
| Control | Untreated | 3.45 | 3.82 | 11.8 | 34 | 144 |
| Test articles | | 2.59-3.64 | 3.64-3.98 | 11.4-12.4 | 35.7-34.1 | 152-183 |

| | | | | | | |
|---|---|---|---|---|---|---|
| * Where, WBC is white blood cells and RBC is red blood cells. | | | | | | |

The test articles tested didn't show any adverse effect on cellular components of human blood, as seen in table 1 when compared to negative and untreated samples. Whereas increase in platelet concentration was observed for test article and this may give an indication of platelet aggregation when in contact with the test articles. The white blood cells were seemed to have been affected by some of the test articles, but this also indicates high compatibility with chitosan.

### Example 4: Unactivated partial thromboplastin time assay (UPTT)

The in-vitro unactivated partial thromboplastin time assay measures the effect of a test article in clotting time of human plasma. The UPTT assay measures plasma factors involved in the generation of plasma thromboplastin and measures the time required to generate thrombin and fibrin polymers via the intrinsic pathway. The test article or its extract is compared to a negative control and a positive control. This test is particularly appropriate to detect activating effects on the coagulation. The test is carried out in triplicate and the results after exposure to the test article are statistically compared to those of the untreated and negative controls (if p≥0.05, the test article passes).

The UPTT was conducted on the hemostatic foams obtained in Example 1. As a positive control, plasma exposed to glass was used. As the negative control, plasma exposed to polyethylene plastic was used.

None of the samples were seen as pro-coagulant when compared to the untreated plasma.

## Claims

1. A hemostatic foam for packing antrums or other cavities of the human or animal body comprising a blend of a chitosan hemostatic agent and a phase-separated polymer that provides the foam with a porosity of 85-99% and a foam density of 0.01-0.2 g/cm³, wherein the foam density is calculated as the polymer mass per volume unit foam,
wherein said phase-separated polymer comprises an amorphous segment and a crystalline segment,
wherein at least said amorphous segment comprises a hydrophilic segment; and
wherein the amount of chitosan hemostatic agent is 5-35 wt. % of the total weight of the foam.

2. The foam according to claim 1, wherein said hemostatic agent is present in said foam in the form of particles.

3. The foam according to claim 2, wherein said particles have a size from 1-1000 µm, preferably 10-90 µm and/or a degree of deacetylation of 1-100 mol %.

4. The foam according to any one of the previous claims, wherein said chitosan hemostatic agent is a chitosan salt, preferably selected from the group consisting of chitosan acetate, chitosan nitrate, chitosan phosphate, chitosan glutamate, chitosan lactate, chitosan citrate, and hydrochloride salts of chitosan.

5. The foam according to any of the previous claims, wherein said phase-separated polymer is of formula (I):
⁅R-Q¹[-R'-Z¹-[R"-Z²-R‴-Z³]ₚ-R"-Z⁴]_{q}-R'-Q²⁆ₙ (I)
wherein R is a polymer or copolymer selected from one or more aliphatic polyesters, polyether esters, polyethers, polyanhydrides, and/or polycarbonates, and at least one R comprises a hydrophilic segment; R', R" and R‴ are independently C₂-C₈ alkylene, optionally substituted with C₁-C₁₀ alkyl or C₁-C₁₀ alkyl groups substituted with protected S, N, P or O moieties and/or comprising S, N, P or O in the alkylene chain; Z¹-Z⁴ are independently amide, urea or urethane, Q¹ and Q² are independently urea, urethane, amide, carbonate, ester or anhydride, n is an integer from 5-500; and p and q are independently 0 or 1.

6. The foam according to any one of the previous claims, wherein said hemostatic agent is distributed at the boundaries of said foam, or homogeneously throughout said foam, or as a gradient within said foam.

7. The foam according to any one of the previous claims, wherein said foam is bioresorbable.

8. The foam according to claim 1, wherein said phase-separated polymer is selected from the group consisting of polyesters, polyhydroxyacids, polyetheresters, polycarbonates, polydioxanes, polyanhydrides, polyurethanes, polyester(ether)urethanes, polyurethane urea, polyamides, polyesteramides, polyorthoesters, polyaminoacids, polyphosphonates, polyphosphazenes, and combinations thereof.

9. The foam according to any one of the previous claims, wherein said phase-separated polymer in said blend has a degree of cross-linking of 0.01 or lower, most preferably about zero.

10. The foam according to any one of the previous claims for use as a nasal dressing.

11. A process for preparing a hemostatic foam according to any one of the previous claims comprising the steps of:
dissolving a phase-separated polymer as defined in claim 1 in a solvent, thus obtaining a polymer solution;
blending a chitosan hemostatic agent with said polymer solution to form a mixture; and
removing said solvent by freeze-drying.

12. The process according to claim 11, wherein said chitosan hemostatic agent is in the form of particles, said process comprising the steps of:
dissolving at least one synthetic polymer as defined in claim 1 in one or more solvents to form a solution;
contacting chitosan hemostatic agent particles with said solution to form a polymer/particles mixture; and
freeze-drying said polymer/ particles mixture by:
freezing said polymer/particles mixture; and
subsequently subliming said one or more solvents to form a synthetic foam comprising said particles.

## Patentansprüche

1. Hämostatischer Schaum zum Füllen von Antrums oder anderen Hohlräumen des menschlichen oder tierischen Körpers, umfassend eine Mischung aus einem hämostatischen Chitosan-Mittel und einem phasengetrennten Polymer, die dem Schaum eine Porosität von 85-99% und eine Schaumdichte von 0,01-0,2 g/cm³ verleiht, wobei die Schaumdichte als die Polymermasse pro Volumeneinheit Schaum berechnet wird,
wobei das phasengetrennte Polymer ein amorphes Segment und ein kristallines Segment umfasst,
wobei zumindest das amorphe Segment ein hydrophiles Segment umfasst; und
wobei die Menge des hämostatischen Chitosan-Mittels 5-35 Gew.-% des Gesamtgewichts des Schaums beträgt.

2. Schaum nach Anspruch 1, wobei das hämostatische Mittel in dem Schaum in Form von Partikeln vorhanden ist.

3. Schaum nach Anspruch 2, wobei die Partikel eine Größe von 1-1000 µm, vorzugsweise 10-90 µm, und/oder einen Deacetylierungsgrad von 1-100 Mol-% aufweisen.

4. Schaum nach einem beliebigen der vorhergehenden Ansprüche, wobei das hämostatische Chitosan-Mittel ein Chitosan-Salz ist, das vorzugsweise aus der Gruppe ausgewählt ist, die aus Chitosan-Acetat, Chitosan-Nitrat, Chitosan-Phosphat, Chitosan-Glutamat, Chitosan-Lactat, Chitosan-Citrat und Hydrochlorid-Salzen von Chitosan besteht.

5. Schaum nach einem beliebigen der vorhergehenden Ansprüche, wobei das phasengetrennte Polymer die Formel (I) aufweist:
⁅R-Q¹[-R'-Z¹-[R"-Z²-R‴-Z³]ₚ-R"-Z⁴]_{q}-R'-Q²⁆ₙ (I)
worin R ein Polymer oder Copolymer ist, ausgewählt aus einem oder mehreren aliphatischen Polyestern, Polyetherestern, Polyethern, Polyanhydriden und/oder Polycarbonaten, und mindestens ein R ein hydrophiles Segment umfasst; R', R" und R‴ sind unabhängig voneinander C₂ -C₈ Alkylen, gegebenenfalls substituiert mit C₁ -C₁₀ Alkyl- oder C₁ -C₁₀ Alkylgruppen, die mit geschützten S-, N-, P- oder O-Einheiten substituiert sind und/oder S, N, P oder O in der Alkylenkette umfassen; Z¹-Z⁴ unabhängig voneinander Amid, Harnstoff oder Urethan sind, Q¹ und Q² unabhängig voneinander Harnstoff, Urethan, Amid, Carbonat, Ester oder Anhydrid sind, n eine ganze Zahl von 5-500 ist; und p und q unabhängig voneinander 0 oder 1 sind.

6. Schaum nach einem beliebigen der vorhergehenden Ansprüche, wobei das hämostatische Mittel an den Grenzflächen des Schaums oder homogen im Schaum oder als Gradient im Schaum verteilt ist.

7. Schaum nach einem beliebigen der vorhergehenden Ansprüche, wobei der Schaum bioresorbierbar ist.

8. Schaum nach Anspruch 1, wobei das phasengetrennte Polymer ausgewählt ist aus der Gruppe bestehend aus Polyestern, Polyhydroxysäuren, Polyetherestern, Polycarbonaten, Polydioxanen, Polyanhydriden, Polyurethanen, Polyester(ether)urethanen, Polyurethanharnstoff, Polyamiden, Polyesteramiden, Polyorthoestern, Polyaminosäuren, Polyphosphonaten, Polyphosphazenen und Kombinationen davon.

9. Schaum nach einem beliebigen der vorhergehenden Ansprüche, wobei das phasengetrennte Polymer in der Mischung einen Vernetzungsgrad von 0,01 oder weniger, am meisten bevorzugt etwa Null, aufweist.

10. Schaum nach einem beliebigen der vorhergehenden Ansprüche zur Verwendung als Nasenverband.

11. Verfahren zur Herstellung eines hämostatischen Schaums nach einem der vorhergehenden Ansprüche, das die folgenden Schritte umfasst:
Auflösen eines phasengetrennten Polymers nach Anspruch 1 in einem Lösungsmittel, wodurch eine Polymerlösung erhalten wird;
Mischen eines hämostatischen Chitosan-Mittels mit der Polymerlösung, um eine Mischung zu bilden; und
Entfernung des Lösungsmittels durch Gefriertrocknung.

12. Verfahren nach Anspruch 11, wobei das hämostatische Chitosan-Mittel in Form von Partikeln vorliegt, wobei das Verfahren die folgenden Schritte umfasst:
Lösen mindestens eines synthetischen Polymers nach Anspruch 1 in einem oder mehreren Lösungsmitteln, um eine Lösung zu bilden;
Inkontaktbringen von hämostatischen Chitosanpartikeln mit der Lösung, um ein Polymer/Partikel-Gemisch zu bilden; und
Gefriertrocknung der Polymer/Partikel-Mischung durch:
Einfrieren der Polymer/Partikel-Mischung; und
anschließendes Sublimieren des einen oder der mehreren Lösungsmittel zur Bildung eines synthetischen Schaums, der die Partikel enthält.

## Revendications

1. Mousse hémostatique pour remplir les antres ou d'autres cavités du corps humain ou animal comprenant un mélange d'un agent hémostatique chitosane et d'un polymère à phases séparées qui confère à la mousse une porosité de 85 à 99 % et une masse volumique de mousse de 0,01 à 0,2 g/cm³, dans laquelle la masse volumique de mousse est calculée comme la masse de polymère par unité de volume de mousse,
dans laquelle ledit polymère à phases séparées comprend un segment amorphe et un segment cristallin,
dans laquelle au moins ledit segment amorphe comprend un segment hydrophile ; et
dans laquelle la quantité d'agent hémostatique chitosane est de 5 à 35 % en poids du poids total de la mousse.

2. Mousse selon la revendication 1, dans laquelle ledit agent hémostatique est présent dans ladite mousse sous forme de particules.

3. Mousse selon la revendication 2, dans laquelle lesdites particules ont une taille de 1 à 1 000 µm, de préférence de 10 à 90 µm et/ou un degré de désacétylation de 1 à 100 % en moles.

4. Mousse selon l'une quelconque des revendications précédentes, dans laquelle ledit agent hémostatique chitosane est un sel de chitosane, de préférence choisi dans le groupe constitué par l'acétate de chitosane, le nitrate de chitosane, le phosphate de chitosane, le glutamate de chitosane, le lactate de chitosane, le citrate de chitosane et les sels chlorhydrate de chitosane.

5. Mousse selon l'une quelconque des revendications précédentes, dans laquelle ledit polymère à phases séparées est de formule (I) :
[-R-Q¹[-R'-Z¹-[R"-Z²-R‴-Z³]ₚ-R"-Z⁴]_{q}-R'-Q²]ₙ (I)
dans laquelle R est un polymère ou un copolymère choisi parmi un ou plusieurs des polyesters aliphatiques, des polyétherester, des polyéthers, des polyanhydrides et/ou des polycarbonates, et au moins un R comprend un segment hydrophile ; R', R" et R'" sont indépendamment un alkylène en C₂-C₈, éventuellement substitué par des groupes alkyle en C₁-C₁₀ ou alkyle en C₁-C₁₀ substitués par des fractions S, N, P ou O protégés et/ou comprenant S, N, P ou O dans la chaîne alkylène ; Z¹ à Z⁴ sont indépendamment un amide, l'urée ou l'uréthane, Q¹ et Q² sont indépendamment l'urée, l'uréthane, un amide, un carbonate, un ester ou un anhydride, n est un nombre entier de 5 à 500 ; et p et q sont indépendamment 0 ou 1.

6. Mousse selon l'une quelconque des revendications précédentes, dans laquelle ledit agent hémostatique est distribué aux limites de ladite mousse, ou de manière homogène dans toute ladite mousse, ou en gradient au sein de ladite mousse.

7. Mousse selon l'une quelconque des revendications précédentes, dans laquelle ladite mousse est biorésorbable.

8. Mousse selon la revendication 1, dans laquelle ledit polymère à phases séparées est choisi dans le groupe constitué par les polyesters, les polyhydroxyacides, les polyétheresters, les polycarbonates, les polydioxanes, les polyanhydrides, les polyu-réthanes, les polyester(éther)uréthanes, les polyuréthaneurées, les polyamides, les polyesteramides, les polyorthoesters, les polyaminoacides, les polyphosphonates, les polyphosphazènes et leurs combinaisons.

9. Mousse selon l'une quelconque des revendications précédentes, dans laquelle ledit polymère à phases séparées dans ledit mélange a un degré de réticulation de 0,01 ou moins, mieux encore d'environ zéro.

10. Mousse selon l'une quelconque des revendications précédentes pour une utilisation comme pansement nasal.

11. Procédé de préparation d'une mousse hémostatique selon l'une quelconque des revendications précédentes comprenant les étapes de :
dissolution d'un polymère à phases séparées tel que défini dans la revendication 1 dans un solvant, obtenant ainsi une solution de polymère ;
mélange d'un agent hémostatique chitosane avec ladite solution polymère pour former un mélange ; et
élimination dudit solvant par lyophilisation.

12. Procédé selon la revendication 11, dans lequel ledit agent hémostatique chitosane est sous forme de particules, ledit procédé comprenant les étapes de :
dissolution d'au moins un polymère synthétique tel que défini dans la revendication 1 dans un ou plusieurs solvants pour former une solution ;
mise en contact des particules d'agent hémostatique chitosane avec ladite solution pour former un mélange de polymère/particules ; et
lyophilisation dudit mélange de polymère/particules par :
congélation dudit mélange de polymère/particules ; et
sublimation ensuite desdits un ou plusieurs solvants pour former une mousse synthétique comprenant lesdites particules.
